# EUROPEAN PATENT APPLICATION

(11) **EP 2 457 704 A1**
(43) Date of publication of application: **30.05.2012**
(21) Application number: 10802187.4
(22) Date of filing: 05.07.2010
(51) Int. Cl.: B26D 7/18, A61F 13/15, A61F 13/49, B26D 1/40

(54) **CUTTING DEVICE FOR ABSORBENT ARTICLE BAND BODY**

(30) Priority: 21.07.2009 JP 2009170089
(71) Applicant: Unicharm Corporation, Ehime 799-0111 (JP)
(72) Inventor: MIYAUCHI, Hideaki, Kanonji-shi Kagawa 769-1602 (JP)
(74) Representative: Peter, Julian
(86) International application number: PCT/JP2010/061728
(87) International publication number: WO 2011/010567

(57) **Abstract**

A cutting apparatus comprises a cutter roll (2) and an anvil roll (3), and a strip-like body of absorbent articles fed between the cutter roll (2) and the anvil roll (3) is cut into cut portions by blades (2e) provided on the cutter roll (2). The cutter roll (2) has circumferential ports formed in the circumferential surface thereof and a side port formed in one side surface thereof. A positive/negative pressure manifold (6) has a positive pressure port and a negative pressure port formed in one side surface thereof. The positive/negative pressure manifold (6) is so fixed that the one side surface thereof faces the one side surface of the cutter roll (2). When the side port is communicated with the negative pressure port, a negative pressure is applied into the circumferential ports causing the cut portion to be held by the cutter roll (2) and when the side port is communicated with the positive pressure port, a positive pressure is applied into the circumferential ports causing the cut portion being held to separate away from the cutter roll (2). A slight gap is formed between the one side surface of the cutter roll (2) and the one side surface of the positive/negative pressure manifold.

## Description

### Technical Field

The present invention relates to a cutting apparatus for a strip-like body of absorbent articles.

### Background Art

A cutting apparatus including a cutter roll and an anvil roll which rotate about the axes of rotation nearly in parallel with each other and in the directions opposite to each other, in which a strip-like body of absorbent articles fed to between the cutter roll and the anvil roll is cut into a product portion and a trim by a blade provided on the cutter roll, wherein the cutter roll is constituted by a holding roll capable of temporarily holding the trim, the holding roll having circumferential ports formed in the circumferential surface thereof, a side port formed in the side surface thereof and a communication passage for communicating the circumferential ports with the side port, wherein the cutting apparatus includes a negative pressure manifold having a negative pressure chamber connected to a negative pressure source, the negative pressure manifold having, in the side surface thereof, a negative pressure port communicated with the negative pressure chamber, and the negative pressure manifold being so fixed that the side surface thereof faces the side surface of the holding roll, wherein the communication and shut-off between the side port of the holding roll and the negative pressure port are changed over accompanying the rotation of the holding roll, and when the side port of the holding roll is communicated with the negative pressure port, a negative pressure is applied into the circumferential ports of the holding roll causing the trim to be held by the holding roll is widely known (see PLT 1).

The above cutting apparatus is provided with sealing means such as packing between the side surface of the holding roll and the side surface of the negative pressure manifold by taking the slidability into account.

### Citation List:

Patent Literature:
   PLT 1: Japanese Unexamined Patent Publication No. 2008-237796

### Summary of Invention:

### Technical Problem

However, the holding roll, i.e., the cutter roll rotates at a high speed and in contact with the sealing means and thus the heat of friction is produced. The cutter roll may gradually expand thermally due to the heat of friction and may deform. The cutter that is deformed may cause defective cutting in which the strip may not be sufficiently cut. If the contact pressure is elevated between the cutter roll and the anvil roll in order to avoid defective cutting, then the life of the blade of the cutter roll may decrease.

### Solution to Problem

According to the present invention, there is provided a cutting apparatus comprising a cutter roll and an anvil roll which rotate about the axes of rotation nearly in parallel with each other and in the directions opposite to each other, in which a strip-like body of absorbent articles fed to between the cutter roll and the anvil roll is cut into a plurality of cut portions by a blade provided on the cutter roll, wherein: either one or both of the cutter roll and the anvil roll are constituted by a holding roll capable of temporarily holding the cut portion, the holding roll having a circumferential port formed in the circumferential surface thereof, a side port formed in the side surface thereof and a communication passage for communicating the circumferential port and the side port with each other; the cutting apparatus comprises a positive/negative pressure manifold having a positive pressure chamber connected to a positive pressure source and a negative pressure chamber connected to a negative pressure source, the positive/negative pressure manifold having, in one side surface thereof, a positive pressure port communicated with the positive pressure chamber and a negative pressure port communicated with the negative pressure chamber, and the positive/negative pressure manifold being so fixed that the one side surface thereof faces the one side surface of the holding roll; the communication and shut-off between the side port of the holding roll and the positive pressure port/negative pressure port are changed over accompanying the rotation of the holding roll, when the side port of the holding roll is communicated with the negative pressure port, a negative pressure is applied into the circumferential port of the holding roll causing the cut portion to be held by the holding roll and when the side port of the holding roll is communicated with the positive pressure port, a positive pressure is applied into the circumferential port of the holding roll causing the cut portion being held to separate away from the holding roll; and a slight gap is formed between the one side surface of the holding roll and the one side surface of the positive/negative pressure manifold that are facing each other.

### Advantageous Effects of Invention

It is possible to suppress the defective cutting that results from the thermal expansion of the holding roll.

### Brief Description of the Drawings

Fig. 1 is a general view of a cutting apparatus illustrating a first embodiment of the present invention.
Fig. 2 is a partly sectional front view of a cutter roll shown in Fig. 1.
Fig. 3 is a side view of the cutter roll shown in Fig. 1.
Fig. 4 is a perspective view of a positive/negative pressure manifold shown in Fig. 1.
Fig. 5 is a partly enlarged section view of the cutter roll and the positive/negative pressure manifold shown in Fig. 1.
Fig. 6 is a partly disassembled view of the cutting apparatus shown in Fig. 1.
Fig. 7 is a schematic perspective view illustrating the operation of the cutting apparatus shown in Fig. 1.
Fig. 8(A) is a partly enlarged sectional view of the cutter roll and the positive/negative pressure manifold illustrating a second embodiment of the present invention.
Fig. 8(B) is a side view of the positive/negative pressure manifold illustrating the second embodiment of the present invention.
Fig. 9 is a side view of the positive/negative pressure manifold illustrating a third embodiment of the present invention.
Fig. 10 is a partly enlarged sectional view of the cutter roll and the positive/negative pressure manifold illustrating a fourth embodiment of the present invention.
Fig. 11 is a partly enlarged sectional view of the cutter roll and the positive/negative pressure manifold illustrating a fifth embodiment of the present invention.
Fig. 12 is a partly enlarged sectional view of the cutter roll and the positive/negative pressure manifold illustrating a sixth embodiment of the present invention.
Fig. 13 is a partly enlarged sectional view of the cutter roll and the positive/negative pressure manifold illustrating a seventh embodiment of the present invention.
Fig. 14 is a partly enlarged view of the cutter roll and the positive/negative pressure manifold illustrating an eighth embodiment of the present invention.
Fig. 15 is a partly enlarged view of the cutter roll and the positive/negative pressure manifold illustrating a ninth embodiment of the present invention.
Fig. 16 is a partly enlarged view of the cutter roll and the positive/negative pressure manifold illustrating a tenth embodiment of the present invention.
Fig. 17 is a schematic perspective view illustrating the operation of the cutting apparatus according to an eleventh embodiment of the present invention.
Fig. 18 is a side view of the positive/negative pressure manifold illustrating a twelfth embodiment of the present invention.

### Description of Embodiments

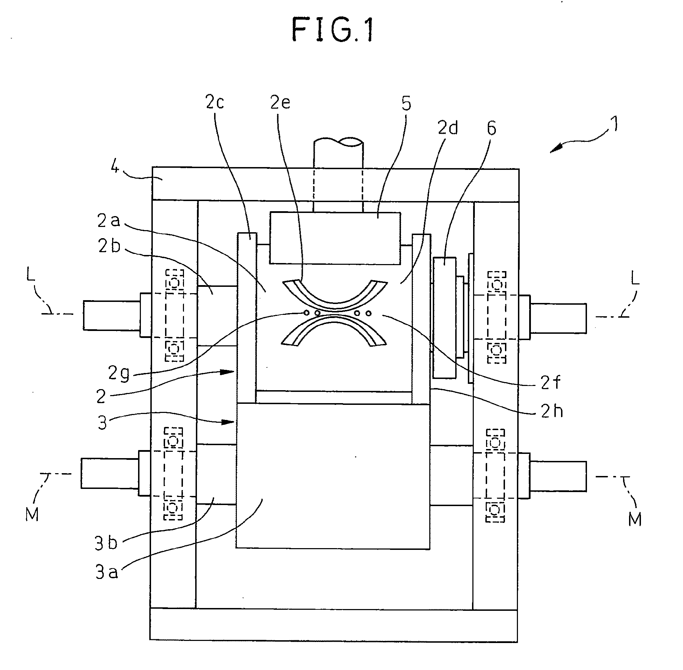

Fig. 1 shows a cutting apparatus 1 according to a first embodiment of the present invention. The cutting apparatus 1 is for cutting a strip-like body of absorbent articles into a plurality of cut portions. According to an embodiment of the present invention, the cut portion comprises an absorbent article and a trim, i.e., the strip is cut into absorbent articles and trims which are then separated. The absorbent article comprises a top sheet, a back sheet and an absorbent body arranged between the top sheet and the back sheet, and is, for example, a sanitary napkin, a panty liner, an incontinence pad or a diaper.

Referring to Fig. 1, the cutting apparatus 1 includes a cutter roll 2 and an anvil roll 3 arranged in the vertical direction. The cutter roll 2 and the anvil roll 3 are supported to rotate about the axes of rotation L and M in nearly the horizontal direction relative to the frame 2, and, therefore, are allowed to rotate about the axes of rotation L and M which are in nearly parallel with each other. The cutter roll 2 and the anvil roll 3 are rotated by a drive unit (not shown) in the directions opposite to each other.

The cutter roll 2 includes a main body 2a and a shaft body 2b. The anvil roll 3, too, includes a main body 3a and a shaft body 3b.

The cutter roll 2 is provided with flanges 2c on both sides of the main body 2a, and the flanges 2c are in contact with the circumferential surface of the main body 3a of the anvil roll 3. Further, the main body 2a is provided on a circumferential surface 2d thereof with, for example, a pair of blades 2e for cutting the strip. A holding region 2f is formed on a region surrounded by the blades 2e on the circumferential surface 2d to temporarily hold a cut portion. A plurality of circumferential ports 2g are formed in the holding region 2f being aligned along, for example, the axis of rotation L.

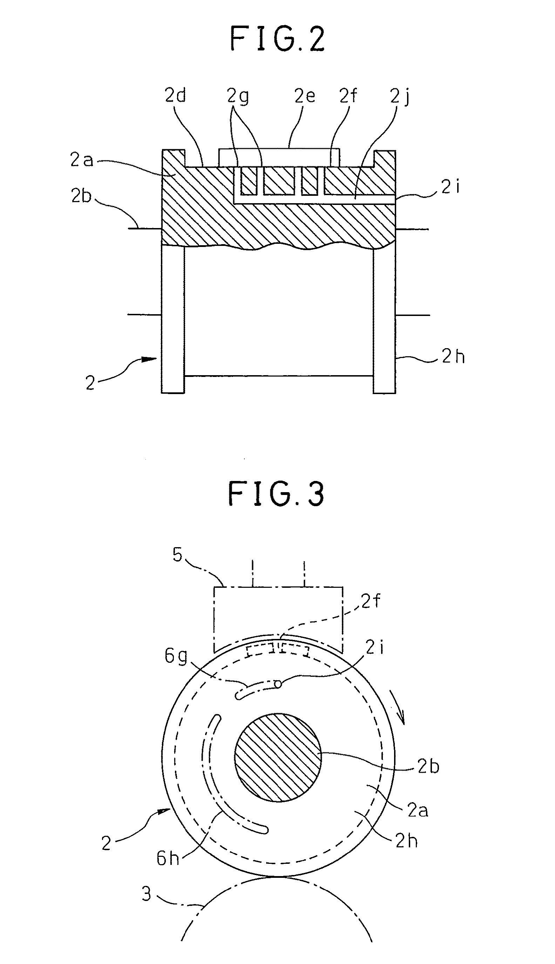

As shown in Figs. 2 and 3, further, a side port 2i is formed in one side surface 2h of the main body 2a of the cutter roll 2. The circumferential ports 2g and the side port 2i are communicated with each other through a communication passage 2j that penetrates through the main body 2a.

Reverting to Fig. 1, a recovery device 5 is arranged over the cutter roll 2 to recover the trims held by the cutter roll 2. The recovery device 5 is connected to a negative pressure source (not shown). Further, a positive/negative pressure manifold 6 is arranged so that it faces the one side surface 2h of the cutter roll 2 while it is not rotatable.

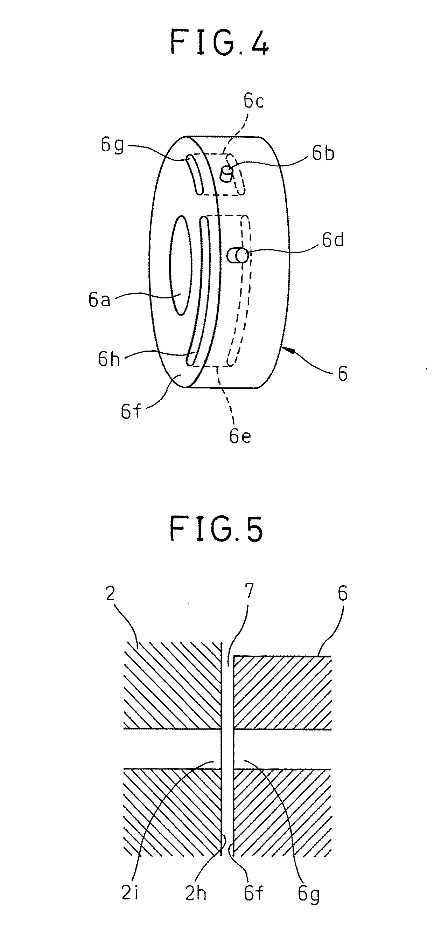

As shown in Fig. 4, the positive/negative pressure manifold 6 has a through hole 6a that penetrates through the center thereof and, therefore, assumes the shape of a ring. The positive/negative pressure manifold 6, further, includes a positive pressure chamber 6c which is connected to a positive pressure source (not shown) at all times through a connection port 6b and a negative pressure chamber 6e which is connected to a negative pressure source (not shown) at all times through a connection port 6d. The positive/negative pressure manifold 6, further, has a positive pressure port 6g of an arcuate shape communicated with the positive pressure chamber 6c and a negative pressure port 6h of an arcuate shape communicated with the negative pressure chamber 6e, the positive pressure port 6g and the negative pressure port 6h being formed in one side surface 6f of the positive/negative pressure manifold 6 facing the one side surface 2h of the cutter roll 2. In this case, as also shown in Fig. 3, the radial positions of the positive pressure port 6g and the negative pressure port 6h from the axis of rotation L are nearly equal to each other, and are also nearly equal to the radial position of the side port 2i of the cutter roll 2 from the axis of rotation L.

When the side port 2i faces the negative pressure port 6h accompanying the rotation of the cutter roll 2, the side port 2i communicates with the negative pressure port 6h, and the negative pressure in the negative pressure chamber 6e is applied into the circumferential ports 2g. Similarly, when the side port 2i faces the positive pressure port 6g, the side port 2i communicates with the positive pressure port 6g, and the positive pressure in the positive pressure chamber 6c is applied into the circumferential ports 2g. On the other hand, as the cutter roll 2 rotates further and the side port 2i no longer faces the negative pressure port 6h, the side port 2i is shut off from the negative pressure port 6h. Similarly, when the side port 2i no longer faces the positive pressure port 6g, the side port 2i is shut off from the positive pressure port 6g. Thus, the communication and shut-off of the side port 2i are changed over relative to the negative pressure port 6h and the positive pressure port 6g accompanying the rotation of the cutter roll 2.

According to an embodiment of the present invention, as will be learned from Fig. 3, too, the negative pressure port 6h is so formed that the side port 2i faces thereto over a rotational angular range in which the holding region 2f comes to face the anvil roll 3 and moves up to arriving at the recovery device 5. Further, the positive pressure port 6g is so formed that the side port 2i faces thereto over a rotational angular range in which the holding region 2f faces the recovery device 5.

As shown in Fig. 5, the positive/negative pressure manifold 6 is so fixed that a slight gap 7 is formed between the one side surface 2h of the cutter roll 2 and the one side surface 6f of the positive/negative pressure manifold 6. Namely, the cutter roll 2 and the positive/negative pressure manifold 6 do not come in contact with each other. In the embodiment shown in Fig. 5, the one side surface 2h and the one side surface 6f are both flat.

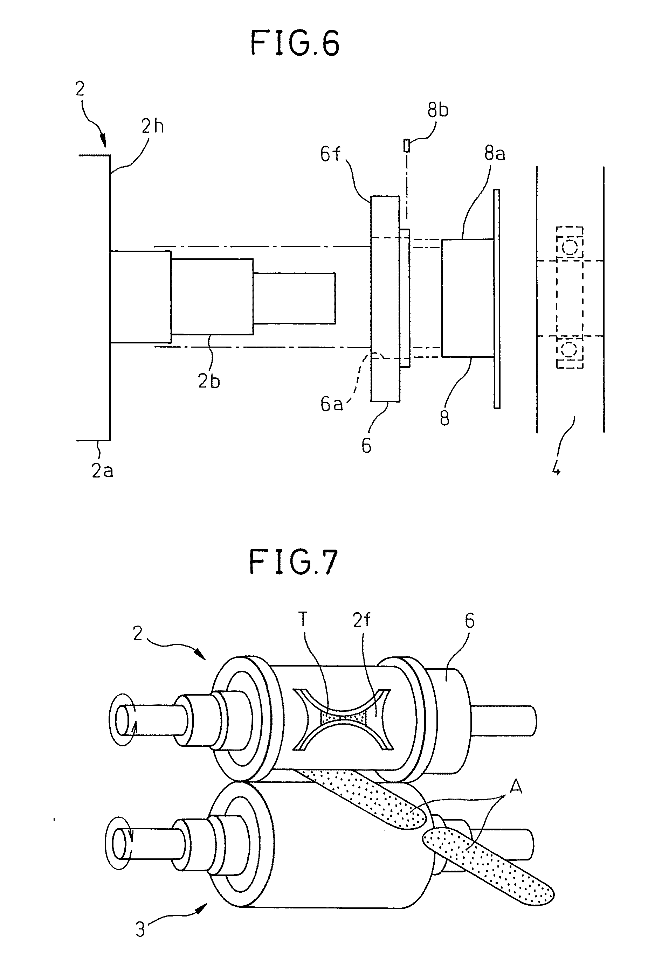

As shown in Fig. 6, a fixing member 8 of a ring-like shape having a cylindrical portion 8a is fixed to the frame 4. The positive/negative pressure manifold 6 is arranged surrounding the cylindrical portion 8a, and is fixed to the cylindrical portion 8a by a screw 8b. The shaft body 2b of the cutter roll 2 penetrates through the through hole 6a of the positive/negative pressure manifold 6 and the through hole of the fixing member 8, and is supported by the frame 4.

In this case, the position of the positive/negative pressure manifold 6 with respect to the fixing member 8 can be adjusted in the direction of the axis of rotation L and, therefore, a magnitude of the gap 7 can be adjusted.

The strip-like body of absorbent articles is conveyed by a conveyer in nearly a horizontal direction, and is fed to the cutting apparatus 1 or, concretely, to between the cutter roll 2 and the anvil rill 3. When the blades 2e reach the strip accompanying the rotation of the cutter roll 2, the strip is cut into an absorbent article A and a trim T as shown in Fig. 7. The absorbent article A is then conveyed by a conveyer (not shown) in nearly a horizontal direction.

While the strip is being cut, on the other hand, the side port 2i of the cutter roll 2 is in communication with the negative pressure port 6h of the positive/negative pressure manifold 6 (see Fig. 3). At this moment, therefore, the negative pressure is applied into the circumferential ports 2g, and the trim T is held in the holding region 2f of the cutter roll 2 due to the negative pressure or the suction force. As the cutter roll 2 rotates further, the trim T is conveyed toward the recovery device 5. When the holding region 2f arrives at the recovery device 5, the side port 2i is shut off from the negative pressure port 6h but is communicated with the positive pressure port 6g (see Fig. 3) whereby the positive pressure is applied into the circumferential ports 2g. As a result, the trim T that had been held by the holding region 2f now separates away from the holding region 2f, and is recovered in the recovery device 5 due to the negative pressure or the suction force of the recovery device 5.

As described above with reference to Fig. 5, the slight gap 7 is formed between the cutter roll 2 and the positive/negative pressure manifold 6. This means that no heat of friction is generated by the contact with the positive/negative pressure manifold 6, and the cutter roll 2 is reliably prevented from being thermally expanded.

Further, the air flow is generated in the gap 7 due to the positive pressure applied into the positive pressure port 6g and the negative pressure applied into the negative pressure port 6h. Namely, the air flowing out from the positive pressure port 6g flows through the gap 7 or the air in the gap 7 flows into the negative pressure port 6h. Therefore, the cutter roll 2 is cooled by the air flow. Accordingly, the cutter roll 2 is further prevented from being thermally expanded. As a result, it is made possible to reliably suppress the defective cutting caused by the cutter roll 2 that is thermally expanded.

The fact that the air flow is generated in the gap 7 means that the positive pressure or the negative pressure is leaking. Therefore, the positive pressure or the negative pressure applied to the circumferential ports 2g may be weakened, and the trim T may not be reliably held or may separate away. It is therefore necessary to suppress the air flow generated in the gap 7 or the leakage.

The air flow in the gap 7 can be suppressed in a manner, for example, as described below.

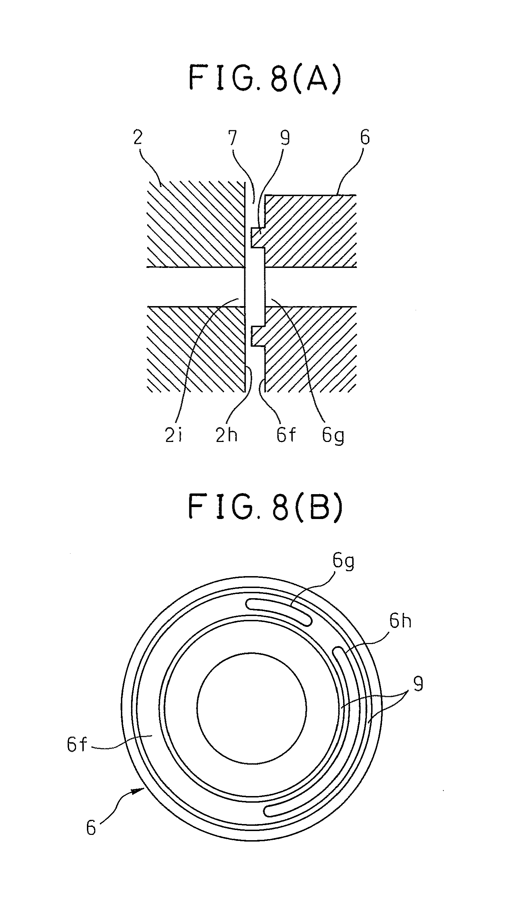

In a second embodiment shown in Figs. 8(A) and 8(B), ring-like projections 9 are formed on one side surface 6f of the positive/negative pressure manifold 6 so as to project toward the one side surface 2h of the cutter roll 2. The projections 9 extend in concentric on the outer side and the inner side in the radial direction so as to surround the positive pressure port 6g and the negative pressure port 6h. As a result, the air flow in the gap 7 can be suppressed. The one side surface 2h of the cutter roll 2 is flat.

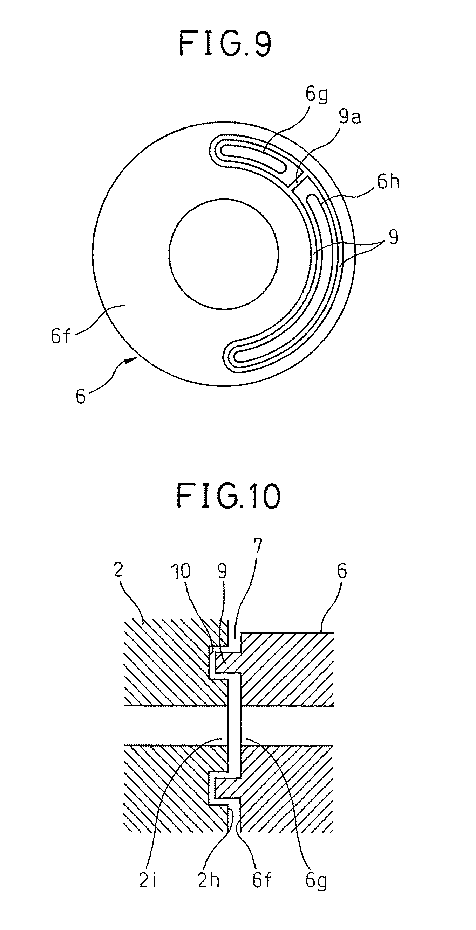

The projections 9 may not be of the shape of a ring. In a third embodiment shown in Fig. 9, the projection 9 extends in an arcuate shape so as to surround the positive pressure port 6g and the negative pressure port 6h. Further, a partition wall 9a is formed between the positive pressure port 6g and the negative pressure port 6h. The partition wall 9a suppresses the air flow from the positive pressure port 6g to the negative pressure port 6h through the gap 7. The projection 9 or the partition wall 9a may be formed on the one side surface 2h of the cutter roll 2, as a matter of course.

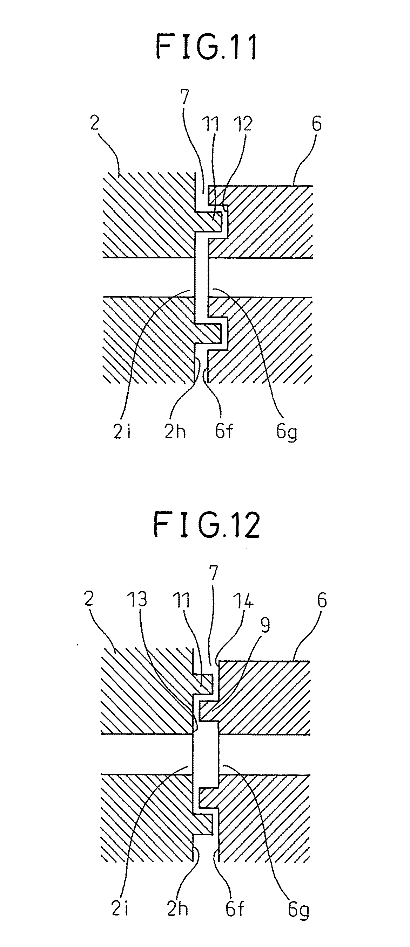

In a fourth embodiment shown in Fig. 10, ring-like recesses 10 are formed in one side surface 2h of the cutter roll 2, and the projections 9 are received in the recesses 10. In a fifth embodiment shown in Fig. 11, projections 11 similar to the projections 9 are formed on one side surface 2h of the cutter roll 2, recesses 12 similar to the recesses 10 are formed in one side surface 6f of the positive/negative pressure manifold 6, and the projections 11 are received in the recesses 12. This enables the positive pressure or the negative pressure to be more reliably applied into the circumferential ports 2g.

In a sixth embodiment shown in Fig. 12, projections 9 are formed on one side surface 6f of the positive/negative pressure manifold 6, and projections 11 are formed on one side surface 2h of the cutter roll 2. Further, the projections 9 are received in a recess 13 defined in one surface 2h on the inner sides of the projections 11, and the projections 11 are received in a recess 14 defined in one side surface 6f on the outer sides of the projections 9.

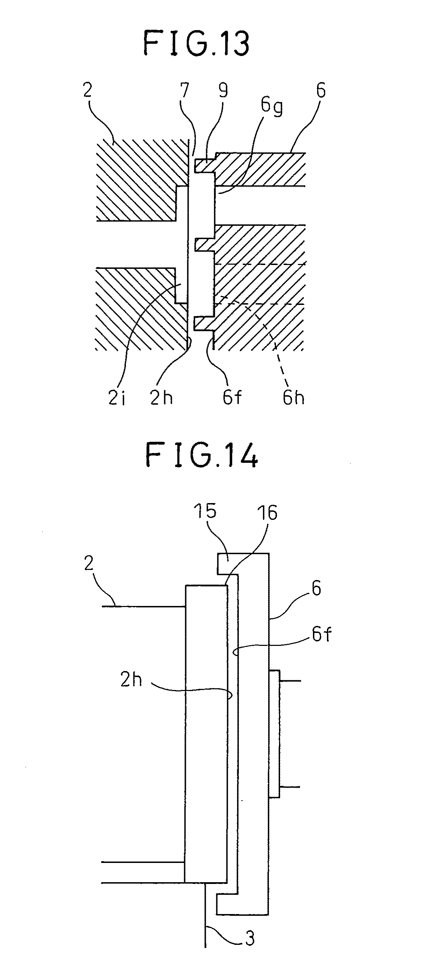

In a seventh embodiment shown in Fig. 13, the radial positions of the positive pressure port 6g and the negative pressure ports 6h are different from each other. In addition, ring-shaped projections 9 are formed on the radially outer side of the positive pressure port 6g, between the positive pressure port 6g and the negative pressure port 6h, and on the radially inner side of the negative pressure port 6h. This suppresses the air flow from the positive pressure port 6g to the negative pressure port 6h, too. In this case, the side port 2i is so dimensioned that it can be communicated with both the positive pressure port 6g and the negative pressure port 6h.

In an eighth embodiment shown in Fig. 14, a ring-like projection 15 is formed along the outer circumferential edge of the one side surface 6f of the positive/negative pressure manifold 6, and an outer circumferential edge 16 of the one side surface 2h of the cutter roll 2 is surrounded by the projection 15. This also makes it possible to suppress the air flow in the gap 7. The projection 15 may not be of the shape of a ring and is, for example, of an arcuate shape.

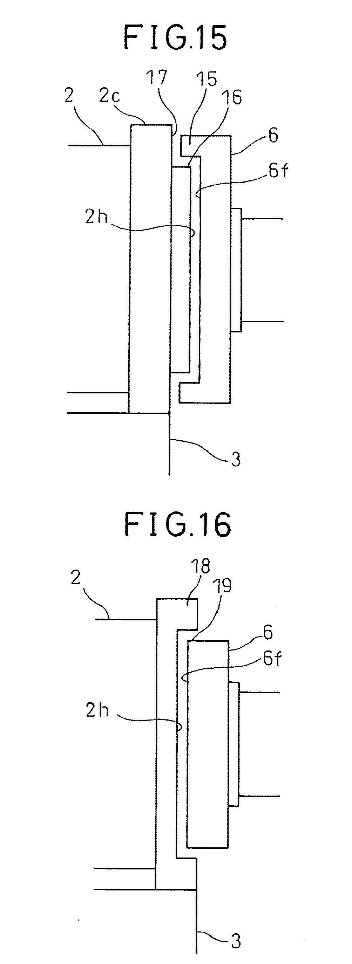

In a ninth embodiment shown in Fig. 15, a ring-like dent 17 is formed in the one side surface 2h of the cutter roll 2 along the outer circumferential edge 16, and the projection 15 is received in the dent 17. The dent 17 can also be formed by attaching a disk member of a reduced diameter to the side surfaces of flanges 2c of the cutter roll 2. In this case, the outer circumferential edge of the disk member constitutes the outer circumferential edge 16 of the one side surface 2h of the cutter roll 2.

In a tenth embodiment shown in Fig. 16, a ring-like projection 18 is formed along the outer circumferential edge of one side surface 2h of the cutter roll 2, and the outer circumferential edge 19 of the one side surface 6f of the positive/negative pressure manifold 6 is surrounded by the projection 18.

In the embodiments of the present invention described above, the trims T are separated from each other. When the absorbent articles A are cut out from the strip the trims T continues like a strip. The present invention can also be applied in this case. In this case, however, the holding regions 2f are formed on the outer circumferential sides of the blades 2e.

In the embodiments of the present invention described above, further, the trim T is held by the cutter roll 2. However, the trim T may be held by the anvil roll 3. Alternatively, either one of the absorbent article A or the trim T may be held by the cutter roll 2 and the other one may be held by the anvil roll 3.

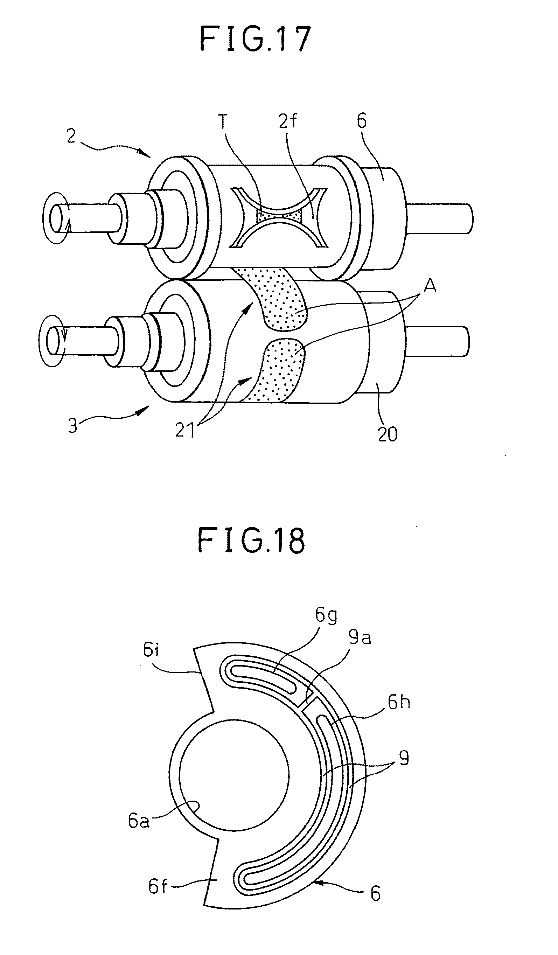

Fig. 17 shows an eleventh embodiment in which the absorbent article A is held by the cutter roll 2 and the trim T is held by the anvil roll 3. A positive/negative pressure manifold 20 is fixed facing the one side surface of the anvil roll 3. A holding region 21 is also formed on the circumferential surface of the anvil roll 3, and the positive pressure or the negative pressure is applied into circumferential ports formed in the holding region 21 from the positive/negative pressure manifold 20. In this case, a slight gap can be maintained between the one side surface of the anvil roll 3 and the one side surface of the positive/negative pressure manifold 20 facing to each other.

Therefore, if a roll capable of temporarily holding the cut portion is referred to as a holding roll, then it can be the that either one or both of the cutter roll 2 and the anvil roll 3 are constituted by the holding rolls. In addition, a slight gap is formed between the one side surface of the holding roll and the one side surface of the positive/negative pressure manifold that are facing each other.

In the embodiments of the present invention described above, further, the positive/negative pressure manifold 6 is assuming the shape of a ring. As shown in Fig. 18 depicting a twelfth embodiment, however, a notch 6i of, for example, a sector shape may be formed in the positive/negative pressure manifold 6 at a position where neither the positive pressure chamber 6c nor the negative pressure chamber 6e has been formed. This permits the one side surface 6f of the cutter roll 2 to be exposed promoting the radiation of heat from the one side surface 6f. Moreover, the positive/negative pressure manifold 6 can be produced using a material in a decreased amount, and the weight thereof can be decreased. The positive/negative pressure manifold 20 can be constituted in the same manner, as a matter of course.

Further, the embodiments of the present invention described above can be combined together. For instance, the projections 9 can be formed on the one side surface 6f of the positive/negative pressure manifold 6, and the projection 15 can be formed along the outer circumferential edge of the one side surface 6f thereof.

### Reference Signs List

- 1: cutting apparatus
- 2: cutter roll
- 2e: blades
- 2g: circumferential ports
- 2h: one side surface of the cutter roll
- 2i: side port
- 3: anvil roll
- 6: positive/negative pressure manifold
- 6f: one side surface of the positive/negative pressure manifold
- 6g: positive pressure port
- 6h: negative pressure port
- 7: gap

## Claims

1. A cutting apparatus comprising a cutter roll and an anvil roll which rotate about the axes of rotation nearly in parallel with each other and in the directions opposite to each other, in which a strip-like body of absorbent articles fed to between the cutter roll and the anvil roll is cut into a plurality of cut portions by a blade provided on the cutter roll, wherein:
either one or both of the cutter roll and the anvil roll are constituted by a holding roll capable of temporarily holding the cut portion, the holding roll having a circumferential port formed in the circumferential surface thereof, a side port formed in the side surface thereof and a communication passage for communicating the circumferential port and the side port with each other;
the cutting apparatus comprises a positive/negative pressure manifold having a positive pressure chamber connected to a positive pressure source and a negative pressure chamber connected to a negative pressure source, the positive/negative pressure manifold having, in one side surface thereof, a positive pressure port communicated with the positive pressure chamber and a negative pressure port communicated with the negative pressure chamber, and the positive/negative pressure manifold being so fixed that the one side surface thereof faces the one side surface of the holding roll;
the communication and shut-off between the side port of the holding roll and the positive pressure port/negative pressure port are changed over accompanying the rotation of the holding roll, when the side port of the holding roll is communicated with the negative pressure port, a negative pressure is applied into the circumferential port of the holding roll causing the cut portion to be held by the holding roll and when the side port of the holding roll is communicated with the positive pressure port, a positive pressure is applied into the circumferential port of the holding roll causing the cut portion being held to separate away from the holding roll; and
a slight gap is formed between the one side surface of the holding roll and the one side surface of the positive/negative pressure manifold that are facing each other.

2. The cutting apparatus for a strip-like body of absorbent articles according to claim 1, wherein a projection is formed on one of the one side surface of the holding roll and the one side surface of the positive/negative pressure manifold, the projection projecting toward the other.

3. The cutting apparatus for a strip-like body of absorbent articles according to claim 2, wherein a recess is formed in the other of the one side surface of the holding roll and the one side surface of the positive/negative pressure manifold, the recess receiving the projection.

4. The cutting apparatus for a strip-like body of absorbent articles according to claim 2, wherein the other of the one side surface of the holding roll and the one side surface of the positive/negative pressure manifold is flat.

5. The cutting apparatus for a strip-like body of absorbent articles according to claim 1, wherein projections are formed in the one side surface of the holding roll and the one side surface of the positive/negative pressure manifold, respectively, the projections projecting toward each other.

6. The cutting apparatus for a strip-like body of absorbent articles according to claim 1, wherein a projection is formed along the outer circumferential edge of one of the one side surface of the holding roll and the one side surface of the positive/negative pressure manifold, the projection surrounding the outer circumferential edge of the other.

7. The cutting apparatus for a strip-like body of absorbent articles according to claim 1, wherein the one side surface of the holding roll and the one side surface of the positive/negative pressure manifold are both flat.

8. The cutting apparatus for a strip-like body of absorbent articles according to claim 1, wherein the positive/negative pressure manifold is so fixed that the gap is adjustable.
